# EUROPEAN PATENT APPLICATION

(11) **EP 3 586 732 A1**
(43) Date of publication of application: **01.01.2020**
(21) Application number: 18758419.8
(22) Date of filing: 19.01.2018
(51) Int. Cl.: A61B 5/0245

(54) **HEART RHYTHM MONITORING APPARATUS**

(30) Priority: 22.02.2017 CN 201710095407
(71) Applicant: Sigknow Biomedical Co., Ltd., Taipei City, Taiwan 10567 (CN)
(72) Inventor: HUNG, Ming-Wei, Taipei City 10567 (TW); WU, Chih-Liang, Taipei City 10567 (TW); CHIU, Yi-Yuan, Taipei City 10567 (TW)
(74) Representative: Scholz, Volker
(86) International application number: PCT/CN2018/073373
(87) International publication number: WO 2018/153188

(57) **Abstract**

Disclosed is a heart rhythm monitoring apparatus, including a water-proof case, a water-proof patch, at least two electrodes, a battery, and an electric circuit. The water-proof case is attached to the water-proof patch to form a water-proof space for accommodating the battery and the electric circuit. Each electrode is connected to the electric circuit, and outwards extends from the water-proof space to form a sensing end. The electrodes penetrate the water-proof patch and the water-proof patch and the sensing ends contact an animal skin. The water-proof patch provides adhesion, and the sensing ends are configured to sense electrical signal on the animal skin. The electrical signal is processed by the electric circuit, and the corresponding heart rhythm information is acquired and recorded. The present invention provides water-proof feature and possesses a simple structure, thereby improving convenience of use, prolonging operation period, acquiring a long-term record of heart rhythm information.

## Description

### CROSS-REFERENCE TO RELATED APPLICATION

This application claims the priority of Chinese patent application No. 201710095407.0, filed on February 22, 2017, which is incorporated herewith by reference.

### BACKGROUND OF THE INVENTION

### 1. Field of the Invention

The present invention relates to a heart rhythm monitoring apparatus, and more specifically to a heart rhythm monitoring apparatus enhanced with water-proof for showering or swimming without taking off, and provided with a simple structure having a shape of a patch, thereby greatly improving convenience in use, prolonging use time, and acquiring and storing a long-term of information about heart rhythm for subsequent detailed diagnosis.

### 2. The Prior Arts

It has been well known that cardiac arrhythmia, that is, abnormality of heart rhythm, may cause various kinds of symptoms like unconsciousness, palpitations, giddiness, or even death. Thus, heart rhythm is commonly used as a key indication of potential heart diseases in the circulatory system. To avoid cardiac arrhythmia and get rid of threat of death, the patient is usually implanted with a pacemaker to regulate heart rhythm. However, the symptoms are often caused by some other minor factors, and particularly, cardiac arrhythmia usually breaks out unexpectedly and abruptly in a short time. Therefore, it has been a topic for the medical personnel to correctly diagnose cardiac arrhythmia from heart rhythm information.

In the prior arts, the electrodes electrically connected to an electronic recorder through connection wires are attached onto the chest for one day. The electronic recorder is configured to save, record, and monitor heart rhythm. However, the shortcoming is that the electrodes need to be replaced every day, and the connection wires are troublesome, inconvenient, and uncomfortable in use.

In addition, the effective operation period of time of the recorder in the prior arts is considerably limited and unable to record heart rhythm for a long time. For example, it needs to take off the electrodes and the recorder when the user does some exercises or daily activities like taking a bath.

Therefore, it is greatly needed to provide a non-invasive heart rhythm monitoring apparatus, which is provided with a simple structure having a shape of a patch, able to improve convenience of use and prolong use time, and particularly enhanced with water-proof such that the user does not need to take off the apparatus even while showering or swimming, thereby overcoming the problems in the prior arts.

### SUMMARY OF THE INVENTION

The primary objective of the present invention is to provide a heart rhythm monitoring apparatus attached onto a skin of an animal or person, comprising a water-proof case, a water-proof patch, at least two electrodes, a battery, and an electric circuit. The water-proof case comprises a first opening and a second opening. The first opening is configured for accommodating a water-proof Universal Serial Bus (USB) socket, which is detachably covered and protected by a dust-free cover. The water-proof USB socket is electrically connected to the electric circuit and for connecting an external USB device. The second opening is intended to accommodate a power on button.

The water-proof patch is attached to a bottom surface of the water-proof case, and provided with a lateral size larger than a lateral size of the water-proof case. A water-proof accommodating space is formed and sandwiched by the water-proof patch and the water-proof case, and the water-proof patch is attached onto the skin. In particular, the electric circuit and the battery are accommodated in the water-proof accommodating space.

The electric circuit in the water-proof accommodating space comprises a circuit board, which is provided with a plurality of circuit elements. The battery in the water-proof accommodating space is turned on by the power on button to supply power to the circuit elements of the electric circuit.

All the electrodes are electrically connected to the electric circuit. Each electrode is provided under the water-proof case, and a part of the electrode outward extends from the water-proof accommodating space to form a sensing end. Also, the sensing end penetrates the water-proof patch and contacts the skin of the animal or person for sensing the electrical signal on the skin corresponding to heart rhythm, and the electric circuit processes the electrical signal to acquire and save long-term information about heart rhythm, thereby implementing the rhythm monitoring function.

Further, the above-mentioned first and second openings are provided on the side or top of the water-proof case. The first opening is for accommodating the eater-proof USB socket to connect the external USB device, and the second opening is for accommodating the power on button for turning on the battery to supply power. Also, the first opening is covered by the dust-free cover to prevent dust from blocking or adversely affecting electrical performance. Moreover, a hollow frame is provided in the water-proof case and has a ring structure with a hollow middle part. The lower part of the hollow frame forms a hook for covering and being inlaid into the electric circuit, and the top part of the hollow frame is configured to carry the battery and further fixed to provide protection for the battery and the electric circuit. Particularly, the hook of the lower part and the circuit board of the electric circuit are configured to meet each other.

The circuit board of the electric circuit is sandwiched between a first buffer layer and a second buffer layer to provide effect of protection and vibration-free for the circuit elements of the circuit board. The battery is provided on a water-proof foam layer, which is provided on the circuit board. Specifically, the battery and the foam layer are provided in the ring structure of the hollow frame.

The water-proof patch comprises an upper release layer, a water-proof film, an enforcement layer, at least two separators, a lower cover, a conductive gel, a first adhesive layer, a second adhesive layer, and a lower release layer, wherein the water-proof film, the enforcement layer, the separators, the lower cover, the conductive gel, the first adhesive layer, and the second adhesive layer are sandwiched between the upper release layer and the lower release layer, and the upper release layer and the lower release layer are previously removed in use.

The upper release layer has a middle opening for surrounding the water-proof case. The water-proof film is under the upper release layer. The enforcement layer is under the water-proof film and has at least two accommodation spaces. Each separator has a ring shape for inserting the accommodation space of the enforcement layer. More specifically, the sensing end of the electrode is provided in the ring shape of the separator. The lower cover is under the enforcement layer and at the middle of the enforcement layer. Also, the lower cover is aligned to the water-proof case for combining together to provide water-proof accommodating space a function of water-proof. Further, the conductive gel is under the sensing end, and thus, the sensing end senses the electrical signal of the skin through the conductive gel so as to prevent the geometrical shape of the sin from affecting the sensitivity of the sensing end.

The first adhesive layer is under the lower cover and has a size equal to or larger than a size of the lower cover, and the second adhesive layer is provided with a ring shape for accommodating the conductive gel. Further, the lower release layer is for carrying the first adhesive layer, the second adhesive layer, and the conductive gel, and part of the water-proof film.

Therefore, the present invention greatly improves convenience of use and employs the water-proof patch attached onto the skin to form a closed subsystem, which prevents the external vapor or water from entering the space between the water-proof patch and the skin so as to prolong the replacing period and the time of the apparatus in use. Furthermore, when the user sweats, the depletion regions formed by the first and second adhesive layers help the present invention to effectively absorb sweat and greatly reduce resistance of perspiration. As a result, the user feels more comfortable while wearing the apparatus of the present invention, the apparatus is well fixed onto the user's body, and the time of wearing is prolonged without risk of detaching.

### BRIEF DESCRIPTION OF THE DRAWINGS

The present invention will be apparent to those skilled in the art by reading the following detailed description of a preferred embodiment thereof, with reference to the attached drawings, in which:
FIG. 1 is a view showing the heart rhythm monitoring apparatus according to the embodiment of the present invention;
FIG. 2 is an exploded perspective view of the water-proof case of the heart rhythm monitoring apparatus of the present invention; and
FIG. 3 is an exploded perspective view of the water-proof patch of the heart rhythm monitoring apparatus of the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

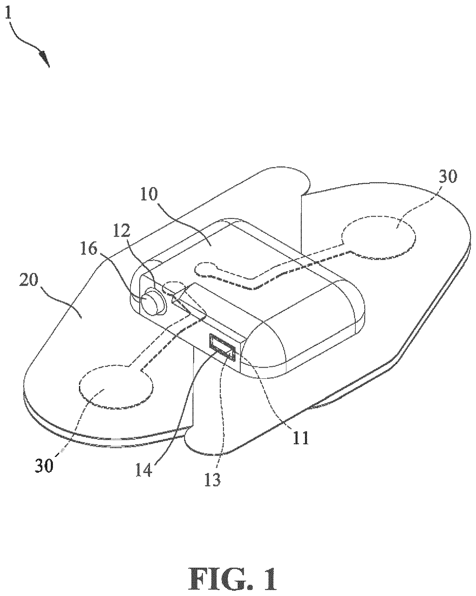

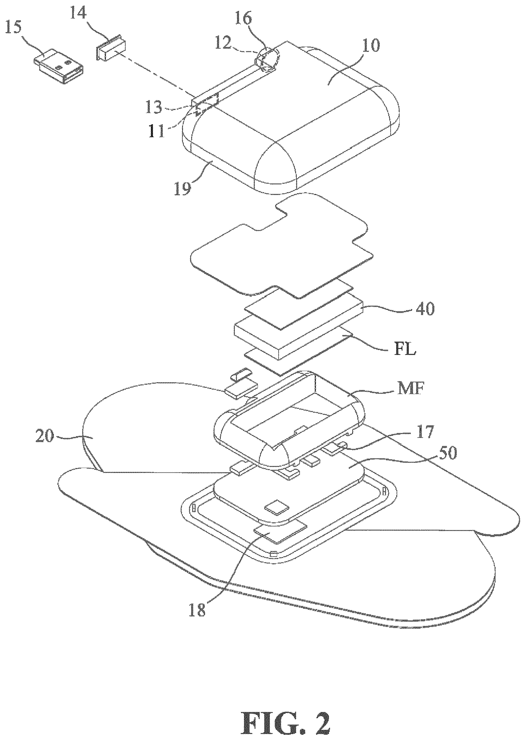

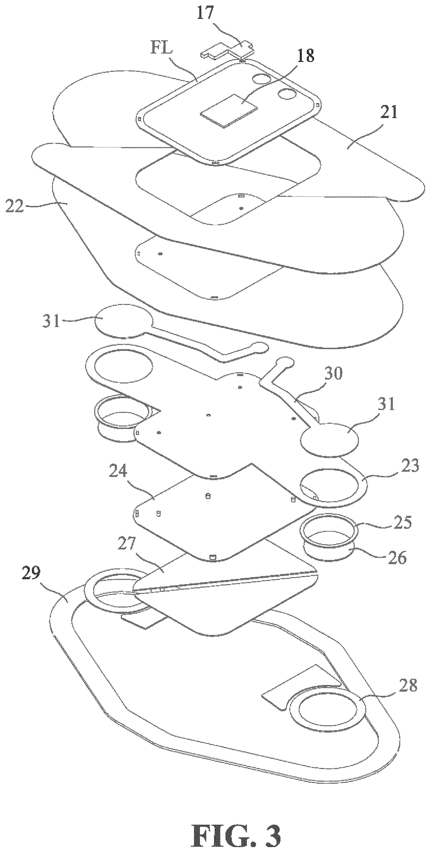

Please refer to FIGs. 1, 2, and 3. Specifically, FIG. 1 illustrates the heart rhythm monitoring apparatus according to the embodiment of the present invention, FIG. 2 is an exploded perspective view of the water-proof case of the heart rhythm monitoring apparatus, and FIG. 3 is an exploded perspective view of the water-proof patch of the heart rhythm monitoring apparatus.

As shown in FIGs. 1, 2, and 3, the heart rhythm monitoring apparatus 1 according to the embodiment of the present invention is configured to be attached onto a skin of an animal or person, and generally comprises a water-proof case 10, a water-proof patch 20, at least two electrodes 30, a battery 40, and an electric circuit 50.

Specifically, the water-proof patch 20 is attached to a bottom surface of the water-proof case 10, and a water-proof accommodating space is formed and sandwiched by the water-proof patch 20 and the water-proof case 10. Further, the water-proof patch 20 outward extends from the internal of the water-proof case 10 to the outer perimeter of the water-proof case 10. In other words, the water-proof patch 20 extends from the bottom of the water-proof case 10 to the outer perimeter of the water-proof case 10 such that the outer perimeter of the water-proof patch 20 forms a continuous and closed contour, and the water-proof patch 20 is attached to the skin. As a result, the water-proof patch 20 and the skin forms a close system, which prevents external vapor from entering. The battery 40 and the electric circuit 50 are accommodated in the water-proof accommodation space. The water-proof case 10 is formed of plastic, metal, silicone, rubber, or carbon fiber. It should be noted that the water-proof patch 20 is provided with a lateral size larger than a lateral size of the water-proof case 10.

Further, each electrode 30 is electrically connected to the electric circuit 50, and configured under the water-proof case 10. Also, the electric circuit 50 is provided with at least two circuit elements, and at least one exposing region of the upper part of each electrode 30 under the water-proof case 10 is connected to the at least two circuit elements to form an electrical connection.

The battery 40 is placed in the water-proof accommodating space, and the electric circuit 50 is also configured in the water-proof accommodating space, and comprises a circuit board 51 for carrying a plurality of circuit elements 52, which are fed power by the battery 40 to operate. The battery 40 is a primary battery or a rechargeable battery for multiple charging like button battery or lithium polymer battery.

Furthermore, each electrode 30 is electrically connected to the circuit board 51, and particularly, the electrode 30 outward extends from the water-proof accommodation space to form a sensing end 31 for contacting the skin. Also, the sensing end 31 penetrates the water-proof patch 20 and contacts the skin for sensing an electrical signal of the skin corresponding to heart rhythm. The electric circuit 50 processes the electrical signal to acquire and save long-term information about heart rhythm or cardiac arrhythmia, thereby implementing the rhythm monitoring function. Moreover, the distance between two adjacent sensing ends respectively contacting the skin is at least 5 cm, and the shape of the two adjacent sensing ends is asymmetric to each other.

More specifically, the water-proof case 10 comprises a first opening 11 and a second opening 12. The first opening 11 is configured for accommodating a water-proof Universal Serial Bus (USB) socket 13, which is detachably covered and protected by a dust-free cover 14 to prevent dust from blocking or adversely affecting electrical performance. Also, the water-proof USB socket 13 is electrically connected to an external USB device 15, and the second opening 12 is intended to accommodate a power on button 16. The battery 40 is turned on by the power on button 16 to supply power to the circuit elements 52.

Further, the water-proof USB socket 13 provided with water-proof is electrically connected to the electric circuit 50 and surrounded by a layer of soft material in collocation with the first opening 11 to form a water tight structure so as to implement water-proof function and prevent vapor from entering the water-proof case 10. Moreover, the water-proof USB socket 13 can be configured to protrude, level, or is lower than the first opening 11. Also, the external USB device 15 like computer or mobile phone is linked to the water-proof USB socket 13 through a USB cable to access internal information of the electric circuit 50. The external USB device 15 in the water-proof space is further electrically connected to the circuit elements 52 of the electric circuit 50 like memory element to access data stored in the memory element.

The above dust-free cover 14 covers the water-proof USB socket 13, and the bottom of the dust-free cover 14 is attached to the water-proof case 10 through the sticky attach layer to prevent dust from entering the water-proof USB socket 13 and affecting data access, or avoid the user carelessly processing USB without permission. Further, the dust-free cover 14 is provided with a pin hole on the side, which is specifically designed to insert a special tool to remove the dust-free cover 14 before data access. As a result, the water-proof USB socket 13 is exposed and ready to access data. On the other hand, it is assured to stop the user carelessly or intentionally removing the dust-free cover 14. Preferably, the dust-free cover 14 is formed of plastic, metal, silicone, rubber, or carbon fiber.

To enhance stability of the circuit board 51 fixed and prevent the circuit elements 52 from abnormal operation due to vibration, the present invention further comprises a first buffer layer 17 and a second buffer layer 18, which are configured to sandwich the circuit board 51 for protection of the circuit elements 52.

The first buffer layer 17 and the second buffer layer 18, which are formed of different plastic, silicone, rubber, or foam material. The first buffer layer 17 has an irregular shape in collocation with the circuit elements 542 of the electric circuit 50. The second buffer layer 18 has a shape equal to or larger than the surface of the circuit elements 52 so as to assure protection for every element.

The water-proof case 10 is also provided with a stop piece 19 at an outer surface of the e water-proof case 10 for preventing the apparatus 1 of the present invention held by a user from skipping due to carelessness.

The apparatus 1 of the present invention further comprises a hollow frame MF provided in the water-proof case 10 to form a ring structure with a hollow middle part. In particular, the hollow frame MF has a lower part forming a hook for covering and being inlaid into the electric circuit 50, and is further configured to fix the battery 40 and the electric circuit 50 for additional protection.

The apparatus 1 of the present invention further comprises a water-proof foam layer FL, which is provided on the circuit board 51, and the battery 40 is provided on the water-proof foam layer FL, further, the battery 40 and the water-proof foam layer FL provided in the ring structure of the hollow frame MF. The water-proof foam layer FL has an area equal to or larger than the area of the battery 40 such that when the user uses the apparatus 1 of the present invention in a normal situation like doing exercise or activity, the water-proof foam layer FL provides buffer effect to greatly alleviate vibration or force due to external impact and further prevent the battery 40 from malfunction.

Further, the water-proof patch 20 comprises an upper release layer 21, a water-proof film 22, an enforcement layer 23, a lower cover 24, at least two separators 25, a conductive gel 26, a first adhesive layer 27, a second adhesive layer 28, and a lower release layer 29. The water-proof film 22, the enforcement layer 23, the lower cover 24, the at least two separators 25, the conductive gel 26, the first adhesive layer 27, and the second adhesive layer 28 are sandwiched by the upper release layer 21 and the lower release layer 29, and the upper release layer 21 and the lower release layer 29 are previously removed when the apparatus is used.

The upper release layer 21 is attached to the water-proof film 22 to provide protection and further enhance stiffness so as to prevent the water-proof film 22 from collapsing and hard attaching the skin. The upper release layer 21 is removed after the apparatus is attached to the skin. Preferably, the upper release layer 21 and the lower release layer 29 are formed of plastic or paper material.

Also, the water-proof foam layer FL is formed of close cell or open cell of foam polymer material. Further, the perimeter of the water-proof foam layer FL is aligned to the bottom perimeter of the water-proof case 10, and the sticky paste layer around the water-proof foam layer FL is employed to combine the water-proof film 22 and the water-proof case 10 to provide water-proof effect for the internal space of the water-proof case 10.

The above upper release layer 21 has a middle opening, which surrounds the water-proof case 10. In other words, the water-proof case 10 can insert into the middle opening.

The water-proof film 22 is under the upper release layer 21 and the water-proof foam layer FL, and outward extends from the internal to the external of the water-proof case 10. The water-proof film 22 is sticky and attached to the skin to form a closed system such that external water and vapor is unable to enter the space between the water-proof film 22 and the skin so as to prolong the attaching period. The water-proof film 22 has a thickness less than 0.1mm, and is ductile, preferably made of water-proof substrate coated with adhesive like a polyurethane (PU) substrate coated with acrylic adhesive. Further, the water-proof film 22 is provided on the first adhesive layer 27 and the second adhesive layer 28, and covers the surrounding region around the first adhesive layer 27 and the second adhesive layer 28.

The at least two electrodes 30 are provided under the water-proof film 22 and in the water-proof case 10, and electrically connected to the electrical circuit 50. The electrodes 50 are configured, arranged, and integrated to the water-proof patch 20, further extending out of the water-proof case 10. The electrodes 30 out of the water-proof case 10 are used to directly or indirectly contact the skin for sensing and transferring the heart rhythm signal to the electrical circuit 50. The electrode 30 is made of silver chloride (AgCl) or silver-silver chloride (Ag/AgCl), or electrically conductive material coated, electroplated, or printed with a layer of AgCl or Ag/AgCl, or a tough material with mechanical strength like rubber, silicone, PU or thermoplastic Polyurethane (TPU) coated, electroplated, or printed with a layer of AgCl or Ag/AgCl served as a signal medium between the skin and the electrically conductive material.

The enforcement layer 23 is under the water-proof film 22 and the electrodes 30, and provided with an adhesive layer on the upper and lower parts of the enforcement layer 23 for upward sticking the water-proof film 22 and the electrodes 30, and at the same time, downward sticking the second adhesive layer 28. The primary effect of the enforcement layer 23 is to enhance mechanical strength of the electrodes 30. The enforcement layer 23 has holes with a number equal to the number of the at least two electrodes 30. For example, two holes serve as two accommodating regions, and are provided out of the water-proof case 10, and in the lap region of the sensing ends 31 of the electrodes 30 and the enforcement layer 23 to accommodate the separators 25 and the conductive gel 26. Additionally, the enforcement layer 23 has an area less than the area of the water-proof film 22, and is made of plastic, silicone, or rubber.

The separators 25 are attached to the electrodes 30 and located in the accommodating regions of the enforcement layer 23. Also, the separator 25 has a partly hollow region for accommodating the conductive gel 26 to separate and prevent the conductive gel 26 and the second adhesive layer 28 from contacting each other, thereby stopping water and substance if any from interchanging or adversely affecting electrical performance.

The separators 25 are made of specific material, which does not or just rarely absorb sweat, like plastic, silicone, rubber, or sponge.

Further, all the separators 25 are under the enforcement layer 23, and each separator 25 has a ring shape for inserting into the accommodation space of the enforcement layer 23, and the sensing end 31 of the electrode 30 is provided in the ring shape of the separator 25.

The conductive gel 26 is electrically conductive and sticky, one side of the conductive gel 26 is under the at least two electrodes 30, and the other side of the conductive gel 26 is intended to contact the skin for serving as a medium to transfer the heart rhythm signal form the skin to the electrodes 30. Particularly, the conductive gel 26 contains little water, resin, and electrolyte.

The lower cover 24 is under the enforcement layer 25 and aligned to the water-proof case 10 for combining each other and implementing water-proof. Further, the top surface of the lower cover 24 is provided with a hook, which is compatible with the slot of the water-proof case 10, such that the whole patch and the water-proof case 10 are tightly attached to demonstrate water-proof property. On the other hand, the lower cover 24 is made of light and tough material like plastic, metal, or rubber. Thus, the electric circuit 50 is stably supported and well protected.

The front side of the first adhesive layer 27 is a sticky attach layer, and the back side of the first adhesive layer 27 is coated with a sticky paste layer for combining the lower part of the lower cover 24. After the sticky attach layer of the first adhesive layer 27 is attached to the skin, the lower cover 24 is prevented from moving or shaking. Thus, the apparatus of the present invention is more stable and well fixed on the user so as to prolong the use time without detaching. The sticky attach layer of the first adhesive layer 27 further has a plurality of depletion regions and is divided into discontinuous segments. Because the user may sweat, the depletion regions are employed to effectively absorb sweat and greatly reduce resistance of perspiration such that the user feels comfortable while wearing the apparatus of the present invention. On the other hand, the sticky attach layer of the first adhesive layer 27 is formed of monomers or a composite of hydrocolloid, polyurethane, silicone, rubber, hot melt glue, or acrylic glue, or made of the above material coated with a base substrate like non-woven fabric or hydrogel as an absorbent substrate, or hydrophilic polymer containing carboxyl base (-COOH) or hydroxyl base (-OH), or a non-absorbent substrate like PU water-proof and vapor permeable film.

Furthermore, the front side of the second adhesive layer 28 is also a sticky attach layer, and the back side of the second adhesive layer 28 is for combining the lower part of the enforcement layer 23. The sticky attach layer of the second adhesive layer 28 is provided with holes corresponding to the enforcement layer 23 for accommodating the separators 25 and the conductive gel 26. Thus, when the sticky attach layer of the second adhesive layer 28 is attached to the skin, the conductive gel 26 is well fixed on the skin without easily moving. Further, the sticky attach layer of the second adhesive layer 28 has a plurality of depletion regions and is divided into discontinuous segments. Because the user may sweat, the depletion regions are employed to effectively absorb sweat and greatly reduce resistance of perspiration such that the user feels comfortable while wearing the apparatus of the present invention. On the other hand, the sticky attach layer of the second adhesive layer 28 is formed of monomers or a composite of hydrocolloid, polyurethane, silicone, rubber, hot melt glue, or acrylic glue, or made of the above material coated with a base substrate like non-woven fabric or hydrogel as an absorbent substrate, or hydrophilic polymer containing carboxyl base (-COOH) or hydroxyl base (-OH), or a non-absorbent substrate like PU water-proof and vapor permeable film.

Specifically, the lower release layer 29 is the lowest layer of the water-proof patch 20 for covering and protecting the adhesive material of the water-proof patch 20 in contact with the skin to avoid pollution. The lower release layer 29 is made of a paper-based substrate coated with a water-proof layer and a release agent or a release layer, or a plastic-based substrate coated with a release agent or a release layer so as to reduce adhesive strength while the lower release layer 29 is removed from the water-proof patch 20, thereby preventing the sticky material from being carried away from the water-proof patch 20.

The lower release layer 29 is further divided into two leaves, and a dust-free protect patch is attached onto the gap between the two leaves such that the user hold the two leaves to easily remove the lower release layer 29 from the inside to the outside, thereby preventing the sticky material from being carried away from the water-proof patch 20.

Overall speaking, the first adhesive layer 27 is under the lower cover 24 and has a size equal to or larger than the size of the lower case 24, and the second adhesive layer 28 is provide with a ring shape for accommodating the conductive gel 26. Additionally, the lower release layer 29 is configured to carry the conductive gel 26,the first adhesive layer 27, and the second adhesive layer 28.

Moreover, the lower perimeter of the water-proof case 10 is specifically configured to tightly combine the lower cover 24 and the foam layer FL so as to form a water-tight structure. The inner wall of the water-proof case 10 is provided with a slot, which is intended to combine the hook of the lower cover 24.

Furthermore, the above electric circuit 50 is arranged between the water-proof case 10 and the water-proof patch 20, and the circuit elements 52 are analog and digital electrical elements, and memory for data storage. More specifically, the electric circuit 50 is responsible for power management, filtration of the cardiac arrhythmia signal sensed, analog-to-digital conversion, and data storage. In addition, the electric circuit 50 further comprises a wireless communication unit like blue tooth, WiFi, or optical communication transmission unit to wireless transmit the cardiac arrhythmia information sensed or acquired to an external system like computer or mobile phone, further, the external system processing and analyzing the information in detail.

From the above mention, one feature of the present invention is that the overall structure of the apparatus is considerably simple and convenient to use, and the user does not need to take the apparatus off even when showering or swimming. In particular, the water-proof patch is attached to the skin to form a closed space such that external water or vapor does not enter the closed space between the water-proof patch and the skin, and the attaching period and the use time are prolonged. In addition, when the user sweats, the present invention employs the depletion region formed by the first and second adhesive layers to effectively absorb sweat and reduce resistance of perspiration. As a result, the user feels comfortable when wearing the apparatus of the present invention, and the apparatus is stable and well fixed on the skin of the user such that it is sure for the user to wear the apparatus for a long time without easily detaching away.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A heart rhythm monitoring apparatus attached onto a skin of an animal or person, comprising:
a water-proof case;
a water-proof patch attached to a bottom surface of the water-proof case, provided with a lateral size larger than a lateral size of the water-proof case, a water-proof accommodating space formed and sandwiched by the water-proof patch and the water-proof case, the water-proof patch attached onto the skin;
an electric circuit provided in the water-proof accommodating space, comprising a circuit board provided with a plurality of circuit elements;
a battery provided in the water-proof accommodating space and electrically connected to the electric circuit for supplying power to the electric circuit; and
at least two electrodes electrically connected to the electric circuit and provided under the water-proof case
wherein a part of the electrode outward extends from the water-proof accommodating space to form a sensing end, each of the sensing ends penetrates the water-proof patch and contacts the skin for sensing an electrical signal of the skin corresponding to heart rhythm, the electrical signal is processed by the electric circuit to acquire and store information of cardiac status for a long period of time, and rhythm monitoring function is implemented.

2. The heart rhythm monitoring apparatus as claimed in claim 1, wherein the water-proof case comprises a first opening for accommodating a water-proof Universal Serial Bus (USB) socket, and the water-proof USB socket is electrically connected to the electric circuit and for connecting an external USB device.

3. The heart rhythm monitoring apparatus as claimed in claim 2, wherein the water-proof USB socket provides a function of water-proof, and is surrounded by a layer of soft material in collocation with the first opening to form a water tight structure to prevent water and vapor from entering the water-proof case.

4. The heart rhythm monitoring apparatus as claimed in claim 1, wherein the water-proof case is provided with a stop piece at an outer surface of the water-proof case preventing the apparatus held by a user from skipping.

5. The heart rhythm monitoring apparatus as claimed in claim 1, further comprising a hollow frame provided in the water-proof case to form a ring structure with a hollow middle part, wherein the hollow frame has a lower part as a hook for covering and being inlaid into the electric circuit, and the hollow frame further fixes the battery and the electric circuit for protection.

6. The heart rhythm monitoring apparatus as claimed in claim 5, further comprising a water-proof foam layer, wherein the foam layer is provided on the circuit board, the battery is provided on the foam layer, and the battery and the foam layer are provided in the ring structure of the hollow frame.

7. The heart rhythm monitoring apparatus as claimed in claim 1, wherein the water-proof case is provided with a power on button, the battery supplies power if the power on button is pressed down, the water-proof case further comprises a second opening for accommodating the power on button.

8. The heart rhythm monitoring apparatus as claimed in claim 1, wherein the water-proof patch comprises an upper release layer, a water-proof film, an enforcement layer, at least two separators, a lower cover, a conductive gel, a first adhesive layer, a second adhesive layer, and a lower release layer, the upper release layer has a middle opening for surrounding the water-proof case, the water-proof film is under the upper release layer, the enforcement layer is under the water-proof film and has at least two accommodation spaces, the at least two separators are under the enforcement layer, each separator has a ring shape for inserting the accommodation space of the enforcement layer, the sensing end of the electrode is provided in the ring shape of the separator, the lower cover is under the enforcement layer and at the middle of the enforcement layer, the lower cover is aligned to the water-proof case for combining together to provide water-proof accommodating space a function of water-proof, the conductive gel is under the sensing end, the first adhesive layer is under the lower cover and has a size equal to or larger than a size of the lower cover, the second adhesive layer is provide with a ring shape for accommodating the conductive gel, the lower release layer is for carrying the first adhesive layer, the second adhesive layer, and the conductive gel, the water-proof film, the enforcement layer, the lower cover, the separators, the conductive gel, the first adhesive layer, and the second adhesive layer are sandwiched between the upper release layer and the lower release layer, and the upper release layer and the lower release layer are removed to use the apparatus.

9. The heart rhythm monitoring apparatus as claimed in claim 8, wherein the first adhesive layer has a front surface and a back surface, the front surface is provided with a sticky attach layer, the back surface is provided with a sticky paste layer, the a sticky paste layer is attached to and combined with a lower part of the lower cover, the sticky attach layer has a plurality of depletion regions and is divided into a plurality of discontinuous segments, and the sticky attach layer is formed of monomers or a composite of hydrocolloid, polyurethane, silicone, rubber, hot melt glue, or acrylic glue.

10. The heart rhythm monitoring apparatus as claimed in claim 8, wherein the second adhesive layer has a front surface and a back surface, the front surface is provided with a sticky attach layer, the back surface is provided with a sticky paste layer, the a sticky paste layer is attached to and combined with a lower part of the enforcement layer, the sticky attach layer is provided with a plurality of holes corresponding to the enforcement layer for accommodating the separator and the conductive gel, the second adhesive layer has a plurality of depletion regions and is divided into a plurality of discontinuous segments, and the sticky attach layer is formed of monomers or a composite of hydrocolloid, polyurethane, silicone, rubber, hot melt glue, or acrylic glue.
